# EUROPÄISCHE PATENTSCHRIFT

(11) **EP 0 705 904 B1**
(45) Veröffentlichungstag und Bekanntmachung des Hinweises auf die Patenterteilung: **04.11.1998**
(21) Anmeldenummer: 95113492.3
(22) Anmeldetag: 28.08.1995
(51) Int. Cl.: C12N 15/74, C12N 1/20, C12N 1/21, G01N 33/00

(54) **Lumineszente nitrifizierende Mikroorganismen**
Luminescent nitrifying microorganisms
Microorganismes nitrifiant et luminescents

(30) Priorität: 08.09.1994 DE 4431964
(43) Veröffentlichungstag der Anmeldung: 10.04.1996
(73) Patentinhaber: BAYER AG, 51368 Leverkusen (DE)
(72) Erfinder: Eberz, Günther, Dr., D-51375 Leverkusen (DE); Rast, Hans-Georg, Dr., D-51465 Bergisch Gladbach (DE)

(56) Entgegenhaltungen:
- FEMS MICROBIOLOGY REVIEWS, Bd. 14, 1994 Seiten 405-414, J.M. COLLARD ET AL. 'Plasmids for heavy metal resistance in Alcaligenes eutrophus CH34: mechanisms and applications'
- FRESENIUS Z. ANAL. CHEM., Bd. 325, 1986 Seiten 136-139, R. KANNE ET AL. 'Analytik bakterientoxischer Effekte mit Hilfe genetisch konstruierter Leuchtbakterien'
- REVIEWS OF ENVIRONMENTAL CONTAMINATION AND TOXICOLOGY, Bd. 125, 1992 Seiten 1-22, B. BITTON AND B. KOOPMAN 'Bacterial and enzymatic bioassays for toxicity testing in the environment'

## Beschreibung

Im Zuge der aeroben oder anaeroben Zersetzung von stickstoffhaltigen organischen Substanzen wird der Stickstoff in Form von Ammonium freigesetzt. Die Umsetzung von Ammonium zu Nitrat, die Nitrifikation, wird sowohl im Boden als auch im Wasser von den nitrifizierenden Bakterien bewirkt. Durch diesen Prozeß wird der globale Stickstoffkreislauf geschlossen: der durch mikrobielle Tätigkeit fixierte elementare und infolge nachgeschalteter Reaktionen organisch gebundene Stickstoff wird nach der o.g. Ammonium-Freisetzung durch die Nitrifikation in Produkte überführt, die durch die sog. denitrifizierenden Bakterien in gasförmige Stickstoffverbindungen recyklisiert werden.

Die Nitrifikanten sind Gram-negative aerobe Bakterien, die zu der Familie der Nitrobacteraceae zusammengefaßt werden. Hierbei arbeiten zwei Bakteriengruppen zusammen: Ammonium wird zunächst durch die Ammoniumoxidanten in Gegenwart von Sauerstoff zu Nitrit oxidiert, das nachfolgend durch die Nitritoxidanten mit Sauerstoff zu Nitrat umgesetzt wird. Die Oxidation von Ammonium oder Nitrit stellt die einzige Energiequelle für die Ammoniakoxidanten bzw. Nitritoxidanten mit Außnahme einiger nitritoxidierender Stämme von *Nitrobacter* dar. Diese Form der Energiegewinnung bezeichnet man als obligate Chemolithotrophie. Der für den Aufbau von Zellsubstanz erforderliche Kohlenstoff wird durch die Fixierung von Kohlendioxid eingeleitet. Es handelt sich daher um autotrophe Bakterien. Die chemolithotrophen Bakterien nehmen bezüglich ihrer Nitrifikationsleistung eine Monopolstellung ein. Die Rate der Nitrifikation durch heterotroph, d.h. mit organischen Verbindungen als Kohlenstoff- und Energiequelle wachsenden Bakterien ist um einen Faktor von 10³ bis 10⁴ kleiner als die der lithoautotrophen Bakterien ("Allgemeine Mikrobiologie", H.G. Schlegel, Georg-Thieme Verlag, 1992). Aufgrund der beschriebenen Oxidationsleistung haben die lithotrophen Bakterien eine große Bedeutung für die biologische Stickstoffeliminierung in Kläranlagen ("Der Einsatz von nitrifizierenden Bakterien zur biologischen Stickstoffentfernung", E. Bock, M. Pohl, M. Bewernick, J. Lorenz, Korrespondenz Abwasser 1/91, Seiten 34-39).

Bekannte und neuentwickelte Testverfahren zur Messung von Hemmstoffen der Nitrifikation

Die nitrifizierenden Bakterien zeigen ein sensitives Verhalten gegenüber verschiedenen organischen Verbindungen ("Nitrification", J.I. Prosser, IRS Press, Oxford-Washington DC, 1986) wie sie auch z.B. in Abwasser vorkommen können. Sie haben vorallem eine ausgeprägte Sensitivität gegenüber Verbindungen wie Allylthioharnstoff oder 2-Chloro-6-trichloromethylpyridin (Nitrapyrin), die in sehr geringen Konzentrationen (Mikromolarbereich) spezifisch die Nitrifikation hemmen ("British Library cataloguing in Publication Data: Nitrification Inhibition in the Treatment of Sewage", M. Richardson, Thames Water Authority, Nugent House, Reading, RG1 8DB, 1985. "Nitrification and Nitrogen Removal", B. Sharma and R.C. Ahlert in: Water Research Vol. 13, Pergamon Press 1977, 897-925). An Verfahren, um die Nitrifikationsleistung zu messen, sind bisher u.a. folgende beschrieben worden: Messung des Ammonium-Verbrauchs mit einer Ammonium-Elektrode, bestehend aus immobilisierten nitrifizierenden Bakterien ("Ammonia electrode with immobilized nitrifiying bacteria", M. Hikuma, T. Kubo, T. Yasuda, I. Karube, S. Suzuki, Anal. Chem. 1980, Seiten 1020-1024). Bestimmung des Sauerstoff-Verbrauchs mit einer Sauerstoffelektrode. Bestimmung des gebildeten Nitrit oder des Nitrat mit einem colorimetrischen Nachweis oder durch HPLC-Technik mit UV-Detektion ("The impact of organic matter on nitric oxide formation by *Nitrosomonas europaea",* R. Stüven, M. Vollmer, E. Bock, Arch. Microbiol. 158, 1992, Seiten 439-443). Bestimmung der Salpetersäure- (Nitrit-) Bildung durch Messung des pH-Wertes. Die Hemmung der Nitrifizierer kann darüber hinaus durch Messungen der Zellzahl oder mittels spezifischer Gensonden für ammoniakoxidierende Bakterien bestimmt werden (EP 045 6047). Um eine Meßmethode zu entwickeln, die in Verbesserung der bisher verfügbaren Verfahren spezifische Hemmstoffe der Nitrifikation innerhalb sehr kurzer Zeit mit hoher Sensitivität und einfacher Automatisierbarkeit anzeigt, stellten wir es uns zur Aufgabe biolumineszente Nitrifizierer herzustellen.

Zur Toxizitätsprüfung von chemischen Verbindungen werden seit vielen Jahren Leuchtbakterien eingesetzt (siehe z.B. "Bioluminescence Assays", A.A. Bulich, CRC Press, Boca-Raton, Florida, USA, 1986 Seiten 57-74). Ein solcher Test beruht darauf, daß bakterientoxische Substanzen zu einer Verminderung der bakteriellen Lumineszenz führen. Dies hängt damit zusammen, daß für die genannte Lumineszenz zelluläre Energie und Reduktionskraft in Form reduzierter Elekronenüberträger erforderlich sind. Bei den natürlichen Leuchtbakterien handelt es sich um eine begrenzte Gruppe von Bakterien, die überwiegend im Meerwasser vorzufinden sind. Natürliche lumineszente nitrifizierende Bakterien sind nicht bekannt.

Der Erfindung liegt die Aufgabe zugrunde, die Vorteile, die eine Toxizitätsmessung mit leuchtenden Teststämmen bietet, auch bei der Nitrifikation auszunutzen.

Dieses Ziel hatte aus zweierlei Gründen wenig Aussicht auf Erfolg: (i) nitrifizierende Bakterien befinden sich aus energetischer Hinsicht in einer äußerst ungünstigen Lage. Ihre Substrate haben ein sehr stark positives Redoxpotential, so daß sich deren Oxidation nicht direkt mit der Reduktion von NAD koppeln läßt. Reduziertes NAD wird jedoch für die Reduktion von Kohlendioxid im Ribulosebisphosphat-Cyclus zum Aufbau von Zellsubstanz benötigt. Zudem ist es auch an der bakteriellen Lumineszenzreaktion involviert. Es gibt experimentelle Hinweise dafür, daß die bei der Substratoxidation auftretenden Elektronen auf der Stufe des Cytochrom c oder a in die Atmungskette eingeschleust werden, was einen entsprechend geringen Energiegewinn und Zellertrag zur Folge hat. Ein Teil dieser Energie wird dazu benutzt, die im Cytochrombereich angelieferten Elektronen über die Atmungskette auf das Niveau der Pyridinnucleotide zurückzuführen und letztere zu reduzieren. Für die nitrifizierenden Bakterien ist der rückläufige Elektronentransport daher ein lebenswichtiger Mechanismus. Nitrifizierende Bakterien wachsen aus den genannten Gründen extrem langsam, wobei Generationszeiten von mindestens 7 Stunden beobachtet werden ("Nitrifikation-die bakterielle Oxidation von Ammoniak zu Nitrat", E. Bock, Forum Mikrobiologie, 1/80). Hierdurch werden sowohl mikrobiologische als auch besonderes genetische Arbeiten mit den Nitrifizierern erschwert. (ii) Nitrifikanten sind bislang genetisch schlecht untersucht. Gentechnisch veränderte Nitrifizierer sind daher noch nicht beschrieben.

### Herstellung lumineszenter nitrifizierender Bakterien

Zur Lösung der obengenannten Aufgabe wurde erfindungsgemäß die Erbinformation für das biologische Leuchten auf obligat chemolithoautotrophe nitrifizierende Mikroorganismen übertragen.

Insbesondere wurden zur Lösung dieser Aufgabe Plasmid- oder Transposonvektoren hergestellt mit deren Hilfe man die genetische Information für das bakterielle Leuchten (*lux*-DNA) in obligat chemolithoautotrophe Nitrifizierer übertragen und ein erfolgreiches Übertragungsereignis sowie ein erfolgtes Ablesen der *lux*-Erbinformation einfach erkennen und die gewünschten gentechnisch veränderten Nitrifizierer selektieren kann. Es wurden dabei Vektorsysteme, bestehend aus einem autonom replizierbaren Weitwirtsbereich-Plasmid -Plasmid oder einem Suizidplasmid mit weitem Wirtsbereich oder einem transposablen Element als Bestandteil eines Suizidplasmids mit weitem Wirtsbereich eingesetzt.

Weitere bevorzugte Ausführungsformen des erfindungsgemäßen Verfahrens sind in den Unteransprüchen 4 bis 7 angegeben.

Die Erfindung betrifft ferner die Verwendung der nach den oben angegebenen Verfahren erhältlichen biolumineszenten, nitrifizierenden Mikroorganismen zur Bestimmung der Vitalität der Mikroorganismen bei einem nitrifizierenden Prozeß durch Messung der Abnahme der Lumineszenz. Bei diesem Meßverfahren werden also die biolumineszenten Mikroorganismen als Indikator für evtl. vorhandene Hemmstoffe eingesetzt. Insbesondere können auf diese Weise Hemmstoffe in einer nitrifizierenden biologischen Kläranlage detektiert werden.

### Ausführungsbeispiele

### Herstellung lumineszenter nitrifizierender Bakterien

Für die Arbeiten wurde exemplarisch eine Reinkultur eines ammoniakoxidierenden Bakteriums aus Abwasser der Bayer AG in Leverkusen isoliert. Es handelt sich hierbei um eine *Nitrosomonas*-Art mit der Bezeichnung RST41-3. Die Zuordnung dieses Stammes erfolgte durch mikrobiologisch physiologische Untersuchungen, sowie durch Einsatz einer DNA-Gensonde, die spezifisch für *Nitrosomonas* ist (EP-0 456 047). Da fraglich war, ob und unter welchen Bedingungen die Fähigkeit der Biolumineszenz in die Ammoniakoxidanten übertragen und in diesen ausgeprägt wird, wurde die Erbinformation der Lumineszenz (*lux*-DNA) mit der genetischen Information für die Disaccharidase β-Galactosidase (lacZ-DNA) in einer Transkriptionsfusion (Operonfusion) gekoppelt. *Nitrosomonas* RST41-3, sowie weitere, eigene Isolate ammoniakoxidierender und nitritoxidierender Bakterien zeigten natürlicherweise keine β-Galactosidase Aktivität. Ein erfolgreiches genetisches Transferereignis und eine Ausprägung der genetischen Information sollte so anhand einer Blaufärbung der Kolonien auf Agarplatten mit 5-Brom-4-chlor-3-indolyl-β-D-galactopyranosid (X-Gal) phänotypisch zu erkennen sein. Gerade bei den extrem langen Inkubaktionszeiten von mehreren Wochen obligat chemolithoautotropher Bakterien, wie den Nitrifizierern ist dies von methodischem Vorteil. So war es zudem möglich, nicht allein nach der vektorvermittelten Antibiotikumresistenz, sondern auch nach Erlangung der β-Galactosidase Aktivität zu selektieren. Somit sollte das mögliche Auftreten spontan resistenter Antibiotikumresistenter Mutanten der gentechnisch zu verändernden Nitrifizierer einfach zu erkennen sein. Auch die aufgrund des geringen Energiegehaltes vermutlich schwach leuchtenden Nitrifizierer können durch die Akkumulation des blauen Farbstoffs prinzipiell erkannt werden.

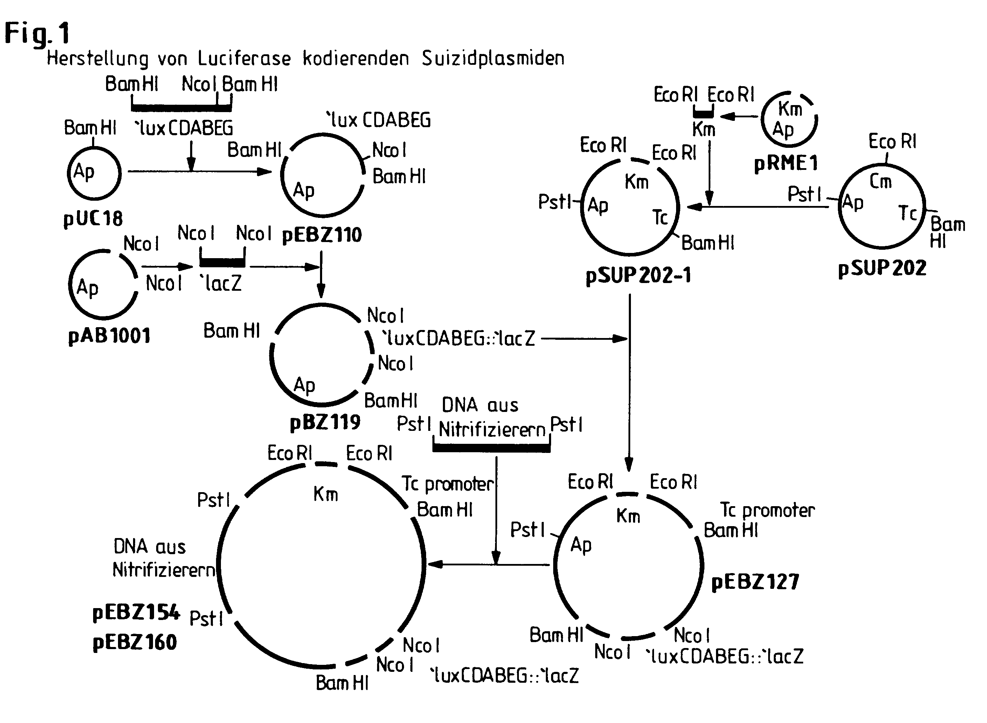

Zur Herstellung von Suizidplasmiden (Abb. 1) wurde der Suizid Weitwirtsbereich Vektor pSUP202 ("A broad host range mobilization system for in vivo genetic engineering: transposon mutagenesis in Gram-negative bacteria", R. Simon, U. Priefer, A. Pühler, Bio/Technology 1, 1983, Seiten 784-791) verwendet. Dieser Vektor vermittelt in *Escherichia coli* Resistenzen gegenüber Ampicillin, Chloramphenicol und Tetracyclin. Nach einem erstellten Antibiogramm von *Nitrosomonas* RST41-3, bot sich keine dieser Resistenzen, vorallem unter der langen und sauren Inkubationsbedingung zur Selektion an. In einem ersten Schritt wurde daher die sich als günstig erwiesene Resitenz gegenüber Kanamycin kloniert. Hierzu wurde das Tn*903*-Kanamycinresistenzgen aus dem Plasmid pRME1 (Dr. W. Messer, MPI für molekulare Genetik, Berlin) das sich in vorherigen Arbeiten als gut handhabbar erwiesen hatte ("Regulation der Hydrogenase-Genexpression und Bildung katalytisch aktiver Hydrogenase in *Alcaligenes eutrophus",* G. Eberz, Dissertation zur Erlangung der Doktorwürde der FU Berlin, 1989) in das Chloramphenicolresistenzgen von pSUP202 kloniert. Das hergestellte Derivat wurde pSUP202-1 genannt.

In einem weiteren Schritt wurde die promotorlose, aber eine Ribosomenbindungsstelle tragende Luciferase-Gensequenz des rechten Luciferase-Operons aus *Vibrio fischeri,* die aus den Genen *luxCDABEG* besteht ("Molecular Biology of Bacterial Bioluminescence", E.A. Meighen, Microbiological Reviews 55, 1991, Seiten 123-142; "Physiological, Biochemical and Genetic Control of Bacterial Bioluminescence", E.A. Meighen and P.V. Dunlap, in: Advances in Microbial Physiology 34, 1993, 1-67) als *Bam* HI-Fragment in den *E*. *coli* Expressionsvektor pUC18 in der Orientierung kloniert, daß die Erbinformation für das Leuchten in entsprechenden Transformanten angeschaltet wurde. Das hergestellte Hybridplasmid erhielt die Bezeichnung pEBZ110. Die Luciferase-DNA entstammte aus dem Transposon Tn*4431* ("Transposon Tn4431 Mutagenesis of *Xanthomonas campestris* pv. *campestris:* Characterization von a nonpathogenic Mutant and Cloning of a Locus for Pathogenicity", J.J. Shaw, L.G. Settles, C.I. Kado, Molecular Plant-Microbe Interactions, 1, 1985, Seiten 3945). Die eingesetzte *lux*-DNA enthält eine *Nco* I Schnittstelle, die sich in dem Gen *luxG* befindet, das selbst für die Ausprägung der Lumineszenz nicht erforderlich ist. Der Vektor pUC18 enthält keine solche Erkennungssequenz. Um die Luciferase-Information mit der genetischen Information für die β-Galactosidase zu fusionieren, war es möglich, eine promotorlose, aber mit einer Ribosomenbindungsstelle versehene *'lacZ*-Kassette zur Insertion in die *Nco* I Schnittstelle von pEBZ110 zu verwenden, wie in dem Plasmid pSUP301-*'lacZ* beschrieben worden ist ("A new family of RSF1010-derived expression and lac-fusion broad-host-range vectors for Gram-negative bacteria", M. Labes, A. Pühler, R Simon, Gene 89, Seiten 37-46, 1990). Für die vorliegende Arbeit wurde eine *'lacZ*-Kassette eingesetzt, die zu beiden Seiten mit einem umfangreicheren Polylinker, bestehend aus gängigen Erkennungsstellen für Restriktionsendonuklease versehen ist. Eine innerhalb des Polylinkers liegende *Nco*I Schnittstelle konnte verwendet werden, die *'lacZ*-Kassette in die oben beschriebene *Nco*I-Schnittstelle von pEBZ110 zu klonieren. Das hergestellte Hybridplasmid wurde pEBZ119 genannt. Es vermittelt in *E*. *coli* Bakterien mit einer Deletion in dem *lacZ*-Gen in Gegenwart von IPTG, dem Induktor des Promotors des verwendeten pUC18 Expressionsvektor sowohl Lumineszenz als auch β-Galactosidase-Aktivtät.

Die *`lux*-*'lacZ*-Transkriptionsfusion wurde als *Bam* HI-Fragment aus pEBZ119 herausgeschnitten und in die *Bam* HI Schnittstelle des Tetracyclinresistenzgen von pSUP202-1 so inseriert, daß der Tetracyclinresistenzgen-Promotor die Expression der Operonfusion initiierte. Das so hergestellte Plasmid pEBZ127 vermittelt in *E*. *coli* sowohl Lumineszenz als auch β-Galactosidase-Aktivität.

In die *Pst* I-Schnittstelle des Ampicillinresistenzgens von pEBZ127 wurden unterschiedliche *Pst* I-Fragmente, isoliert aus der Gesamt-DNA von *Nitrosomonas* RST41-3 eingebaut. Zwei der so erhaltenen Klone erhielten die Bezeichnung pEBZ154 (trägt ein ca. 2,7 kb großes *Nitrosomonas* DNA-Fragment) und pEBZ160 (enthält ein ca. 10 bis 11 kb großes *Nitrosomonas* DNA-Fragment). Dieser Schritt ist erforderlich, da das Basisreplikon dieser Plasmide in *E. coli* vermehrbar und in verschiedene Gram-negative Bakterien übertragbar, aber in letzteren nicht replizierbar ist. Die übertragenen Hybridplasmide sollten jedoch durch einen zellulären DNA-Rekombinationsmechanismus des Bakterienwirtes unter Einbeziehung der klonierten wirtshomologen DNA ins Genom des Ammoniakoxidanten eingegliedert werden.

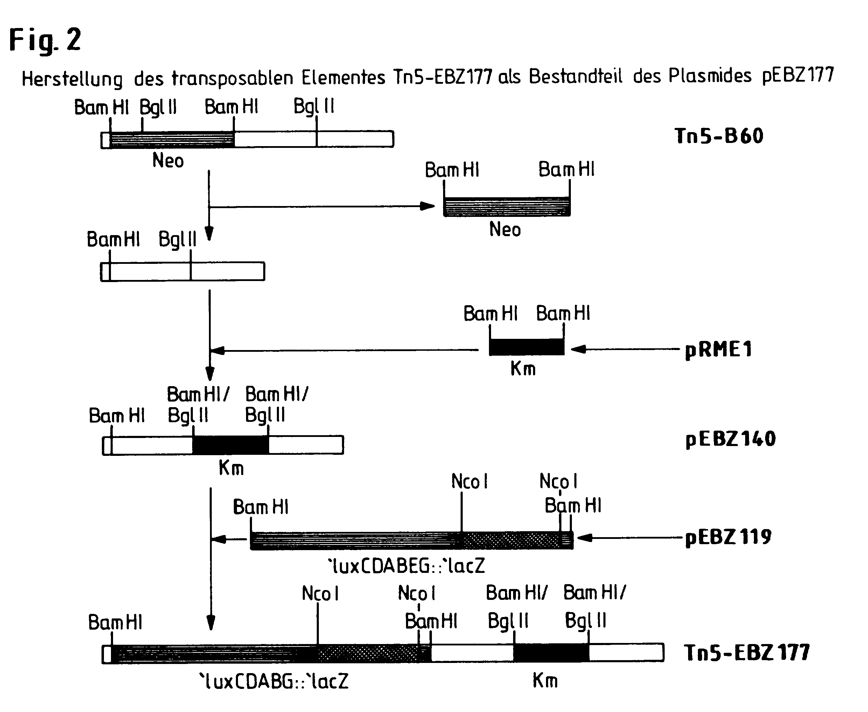

Bei der Konstruktion des transposablen Elementes (Abb. 2) wurde von dem Tn*5*-Derivat B60 ("New derivatives of transposon Tn*5* suitable for mobilization of replicons, generation of operon fusions and induction of genes in Gram-negative bacteria", R. Simon, J. Quandt, W. Klipp, Gene 80, 1989, Seiten 161-169) ausgegangen. Hierzu wurde zunächst das Neomycinresistenzgen einschließlich eines auswärts gerichteten *tac*-Promotors, welche sich beide auf einem *Bam* HI-Fragment des Transposonanteils von pSUP-Tn*5*-B60 befinden, deletiert. In die *Bgl* II-Schnittstelle des so modifizierten Transposons wurde das Kanamycinresistenzgen aus pRME1 (s.o.) als *Bam* HI-Fragment kloniert; es kann durch *Bam* HI-Restriktion aus dem entstandenen Konstrukt (pEBZ140) nicht mehr herausgeschnitten werden. In die durch obige Deletion erzielte singuläre *Bam* HI-Erkennungssequenz wurde die *'lux*-*'lacZ*-Fusion aus pEBZ119 so inseriert, daß die Transkription vom linken Ende des Transposons herkommend initiiert werden kann. Das so hergestellte Transposonplasmid pEBZ177 besteht demzufolge aus einem *'lux-'lacZ*-Promotorsuch-Transposon, das sich in einem Suizid Weitwirtsbereich Vektor befindet.

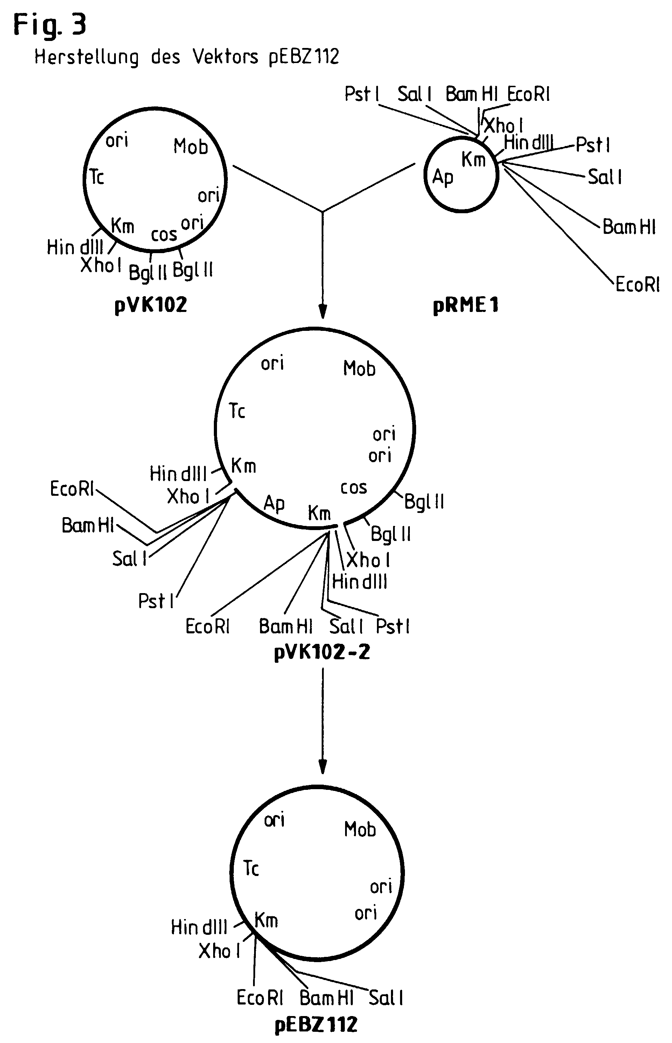

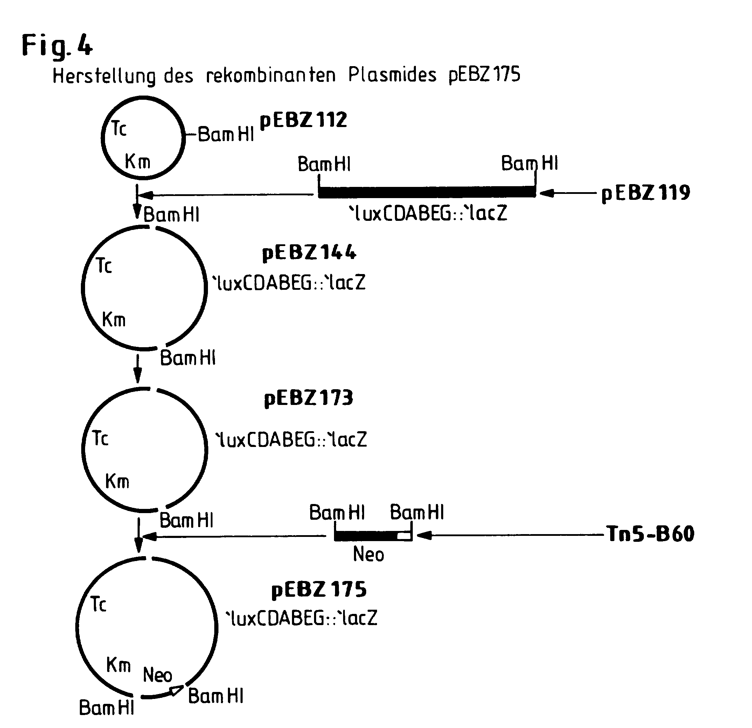

Die Herstellung des, in verschiedenen Gram-negativen Bakterien replizierbaren *'lux*-*'lacZ*-Vektors wurde folgendermaßen durchgeführt (Abb. 3 und 4): in einem ersten Schritt wurde der Cosmid-Vektor pVK102 ("Wide host range cloning vectors: a cosmid clone bank of an *Agrobacterium* Ti plasmid", V.C. Knauf, E.W. Nester, Plasmid 8, 1982, Seiten 45-54) mit *Xho*I linearisiert und mit dem *Xho*I geschnittenen Plasmid pRME1 ligiert (Abb. 3). Das Plasmid pPME1 besteht aus pBR322, das eine Kanamycinresistenzgen-Kassette, die zu beiden Seiten mit einem Polylinker begrenzt ist, in der *Hin*dIII Schnittstelle des Tetrazyklin-Resistenzgens enthält. Dieser Polylinker enthält mehrere Erkennungsstellen für Restriktionsendonukleasen, wie z.B. *Eco* RI, *Bam* HI, *Sal* I oder *Pst* I (Abb. 3). Eines der erhaltenen Ligationsprodukte (pVK102-2) wurde mit *Bgl* II und *Pst* I doppeltgeschnitten und das erhaltene größte Verdauungsprodukt isoliert und nach Vorbehandlung mit Klenow Polymerase religiert. Auf diese Weise wurde der überwiegende pRME1-Anteil sowie die *cos*-site aus dem Kointegrat eliminiert, wobei der so hergestellte Vektor (pEBZ112), direkt angrenzend an das Kanamycinresistenzgen eine kleine multiple cloning site (mcs) trägt. Das Kanamycinresistenzgen in pEBZ112 setzt sich durch diese Vorgehensweise sowohl aus Teilen des Kanamycinresistenzgens aus pVK102 als auch aus Teilen der Kanamycinresistenzgen-Kassette aus pRME1 zusammen. Dies mcs enthält u.a. eine singuläre *Bam* HI-Schnittstelle (Abb. 4), in die nun die *'lux-'lacZ-*Fusion als *Bam* HI-Fagment inseriert werden konnte (pEBZ144). Dieser Vektor wurde unvollständig mit *Bam* HI geschnitten, mit Klenow Polymerase aufgefüllt und religiert, um die am Ende des *'lux*-*'lacZ*-Operons liegende *Bam* HI-Schnittstelle zu zerstören. Der durch diese Methode hergestellte Vektor pEBZ173 trägt nur noch eine *Bam* HI-Erkennungssequenz, die in der mcs vor dem *'lux-'lacZ*-Operon liegt. In diese singuläre *Bam* HI-Stelle wurde das *Bam* HI-Fragment aus dem Suizid-Transposon Plasmid pSUP-Tn*5*-B60 (s.o.), das das Neomycinresistenzgen, sowie den auswärts gerichteten *tac*-Promotor träg, in der Weise kloniert, das der *tac*-Promotor die Transkription der *'lux*-*'lacZ*-Fusion initiiert. Letzteres Plasmid (pEBZ175) vermittelt entsprechenden *lacI*^{*q*} *E. coli* Bakterien die mit IPTG induzierbare Fähigkeit zur Lumineszenz und β-Galactosidase Aktivität.

In einem parallel durchgeführten Ansatz wurde das gesamte *lux*-Regulon aus *V*. *fischeri*, bestehend aus den Regulationsgenen *luxR* und *I* sowie den o.g. *lux*-Strukturgenen als *Sal* I-Fragment in den Vektor pVK102 kloniert. Die *lux*-DNA stammte aus dem rekombinanten Plasmid pJE202 ("Identification of genes and gene products necessary for bacterial bioluminescence", J. Engebrecht and M. Silverman, Proc. Natl. Acad. Sci. USA, 1984, Seiten 4154- 4158). Das hergestellte Plasmid erhielt die Bezeichnung pEBZ145.

### Genetischer Transfer der 'lux-'lacZ-Fusionskonstrukte in Nitrosomonas sp. RST41-3

Die hergestellten Luciferase kodierenden Plasmide konnten, da sie sich in Vektoren mit einer weiten Mobilisierbarkeit in verschiedene Gram-negative Bakterien befinden, in einer Zwei-Eltern-Kreuzung mit dem *Escherichia coli* Stamm S17-1 als Donor ("A broad host range mobilization system for in vivo genetic engineering: transposon mutagenesis in Gram-negative bacteria", R. Simon, U. Priefer, A. Pühler, Bio/Technology 1, 1983, Seiten 784-791) nach *Nitrosomonas* RST41-3 mobilisiert werden. Hierbei wurden folgende Konjugations-Bedingungen gewählt: als nichtselektiver Schritt wurde eine Inkubation von 80 µl einer gut gewachsenen und gewaschenen Kultur des *Nitrosomonas* Rezipienten, eingestellt auf ca. 10¹⁰ Zellen pro ml, zusammen mit 80 µl des *E. coli* Donors, eingestellt auf 10⁶ Zellen pro ml zwei Tage bei 28°C auf einem festen Minimalmedium gewählt. Ein Medium wie in der Publikation "Classification of eight new species of ammonia-oxidizing bacteria: "*Nitrosomonas communis* sp. nov., *Nitrosomonas ureae* sp. nov., *Nitrosomonas aestuarii* sp. nov., *Nitrosomonas marina* sp. nov., *Nitrosomonas nitrosa* sp. nov., *Nitrosomonas eutropha* sp. nov., *Nitrosomonas oligotropha* sp. nov. and *Nitrosmomonas* halophila sp. nov." (H.-P. Koops, B. Böttcher, U.C. Möller, A. Pommerening-Röser, G. Stehr, Journal of General Microbiology 137, 1991, Seiten 1689-1699) angegeben, war dabei anwendbar. Zusätzlich wurde dem Medium die für den *E. coli* Donor notwendige Kohlenstoffquelle in Form von 0.05 % Glucose sowie Prolin und Thiamin zugesetzt. Die Kreuzungspartner wurden mit Minimalmedium ohne Glucose, Prolin und Thiamin abgeschwemmt und geeignete Verdünnungsreihen auf Selektivplatten ausgespatelt. Die Selektivplatten bestanden aus dem o.g. Minimalmedium ohne Glucose, Prolin und Thiamin, aber mit Nalidixinsäure in einer Konzentration von 50 µg/ml und Kanamycin in einer Konzentration von 15 µg/ml sowie X-Gal. Der verwendete *Nitrosomonas* Stamm zeigt eine ausgeprägte Resistenz gegenüber Nalidixinsäure. Nur plasmidtragende Abkömmlinge dieses Stammes verfügen auch über die plasmidkodierte Resistenz gegen Kanamycin. Nach etwa zwei Monaten traten *Nitrosomonas* Transkonjuganten auf. Die erfolgreiche Transkription der *'lux*-Sequenz, sowie die Expression der ß-Galactosidase in den *Nitrosomonas*-Wirten nach Transfer der *lux*-*lacZ*-Fusion war nach etwa zwei Monaten durch eine Blaufärbung der kleinen *Nitrosomonas*-Kolonien zu sehen, die in gewohnter Weise zu einer Ansäuerung des Mediums (Farbumschlag des pH-Indikators Phenolrot, 10 mg/l) führten.

Alle Transkonjuganten Kolonien nach Transfer der Suizidplasmide pEBZ154 und pEBZ160 sowie nach Übertragung des Plasmides pEBZ175 waren auf Indikatorplatten blau gefärbt. Hingegen traten nach Transfer des Plasmides pEBZ177 unter den isolierten Kolonien nur wenige blau verfärbte Transkonjuganten Kolonien auf. Dies spricht dafür, daß das in pEBZ177 vorliegende Tansposon zunächst in das *Nitrosomonas* Genom transponieren mußte, damit die Erbinformation für β-Galactosidase abgelesen werden kann. Hierbei muß die Transposition so erfolgt sein, daß ein *Nitrosomonas* eigener Promotor zum Anschalten der *lac*Z-Information diente.

Das Auftreten dieser ß-Galactosidase ausprägenden Transkonjuganten belegt erstmals, daß de*r E. coli* Konsensus *tac*-Promotor, sowie der Promotor des Tetracyclinresistenzgens aus pEBZ154 und pEBZ160, der von pMB9 abstammt ("Gene und Klone", E.-L. Winnacker, Verlag Chemie, 1984, Seiten 115-119) in einem obligat chemolithoautotrophen Bakterium wie *Nitrosomonas* funktionsfähig ist. Die Tatsache, daß nach Transfer des *'lux*-*'lacZ*-Tn*5*-Derivates in *Nitrosomonas*, neben kanamycinresistenten auch β-Galactosidase ausprägende Abkömmlinge auftraten, demonstriert, daß das hergestellte Tn*5*-Derivat in dem obligaten Chemolithotrophen transponierte.

Die Transkonjuganten nach Transfer des *lux*-Plasmides pEBZ145 waren lediglich anhand ihres kanamycinresistenten Phänotyps auf dem Selektivmedium als rekombinante Klone zu erkennen.

### Hemmungsmessungen mit lumineszenten Nitrifizierern

Die hergestellten blauen Transkonjuganten und die Nitrosomonas RST41-3/pEBZ145 Transkonjuganten wurden auf Selektivmedium gereinigt, in 20 ml flüssigem Selektivmedium ohne X-Gal angeimpft und mehrere Tage bei 28°C kultiviert. Nach ersichtlichem Wachstum wurden mit dieser Vorkultur 200 ml des gleichen Mediums inokuliert und in entsprechender Weise bebrütet.

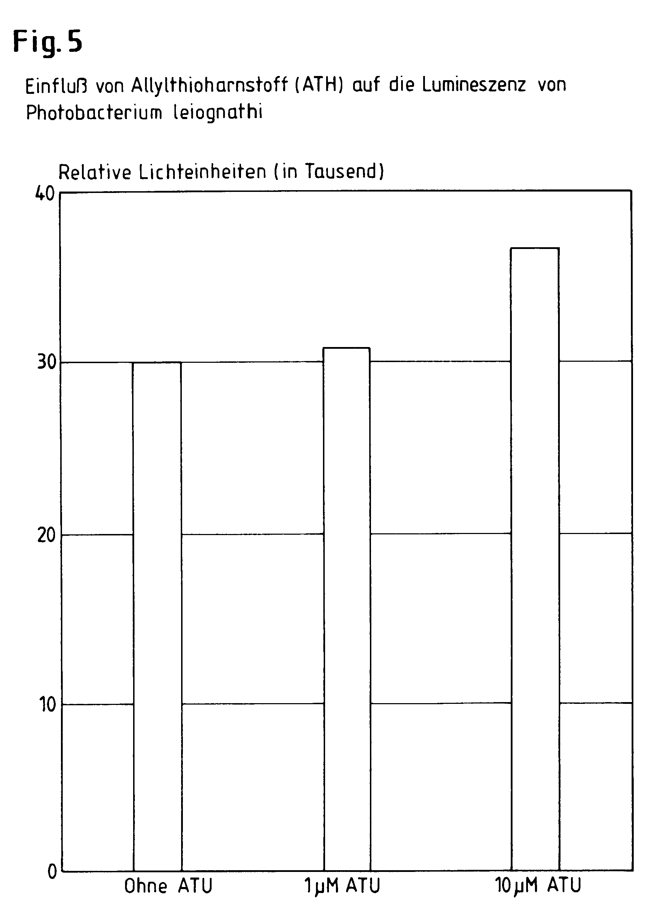

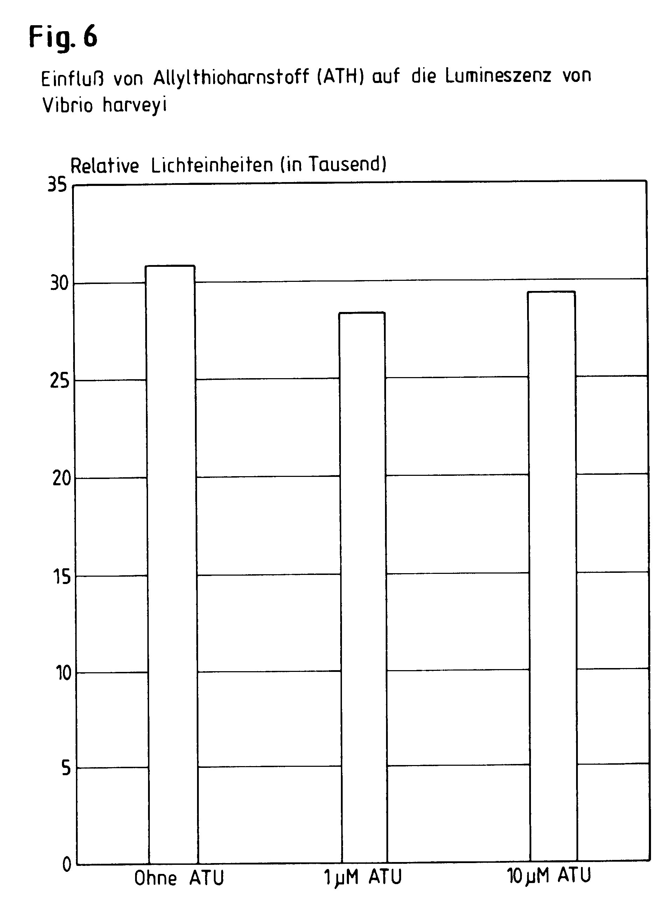

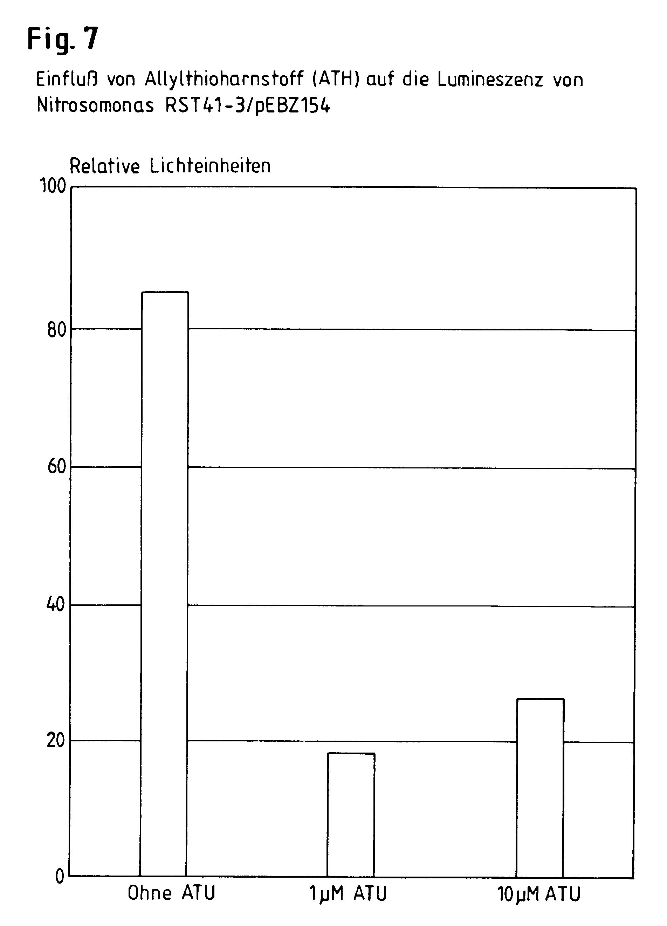

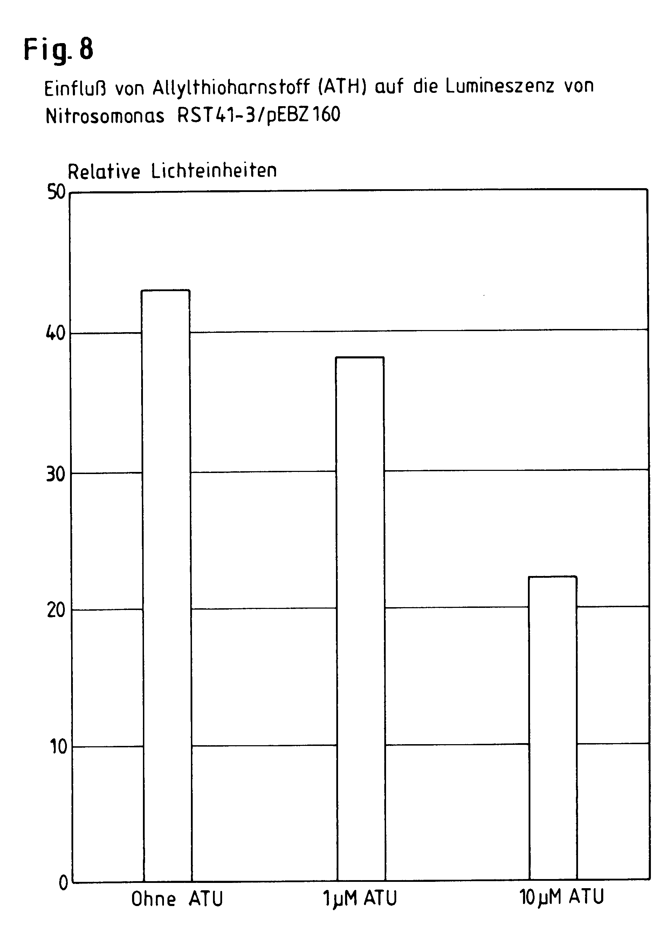

Alle hergestellten *Nitrosomonas* Derivate zeigten eine autonome Lumineszenz. Für die Lumineszenzmessungen wurden die Isolate in der stationären Wachstumsphase geerntet, etwa zweihundertfach in frischem Minimalmedium ohne Nalidixinsäure und Kanamycin konzentriert und 0,1 bis 0,2 ml zur Lumineszenzmessung (Lumac/3M-Röhrchenluminometer Biocounter M2010A; Leerwert RLU: 10 - 12) für 60 sec eingesetzt. Diese war im Vergleich zu den natürlichen Leuchtbakterien *Photobacterium leiognathi* (ATCC 33469) und *Vibrio harveyi* (ATCC14126) nach Anzucht in 246-Medium (Medium nach "Catalogue of Strains 1993", Deutsche Sammlung von Mikroorganismen und Zellkulturen GmbH, Braunschweig) und Messung von 60 sec bedeutend geringer (Abb. 5 und 6). Aber im Gegensatz zu letzteren, ließ sich ihre Lumineszenz, wie exemplarisch für die Stämme *N*. RST41-3/pEBZ154 und *N*. RST41-3/pEBZ160 veranschaulicht, durch die Gabe geringer Konzentrationen des Nitrifikationshemmers Allylthioharnstoff deutlich vermindern (Abb. 7 und 8).

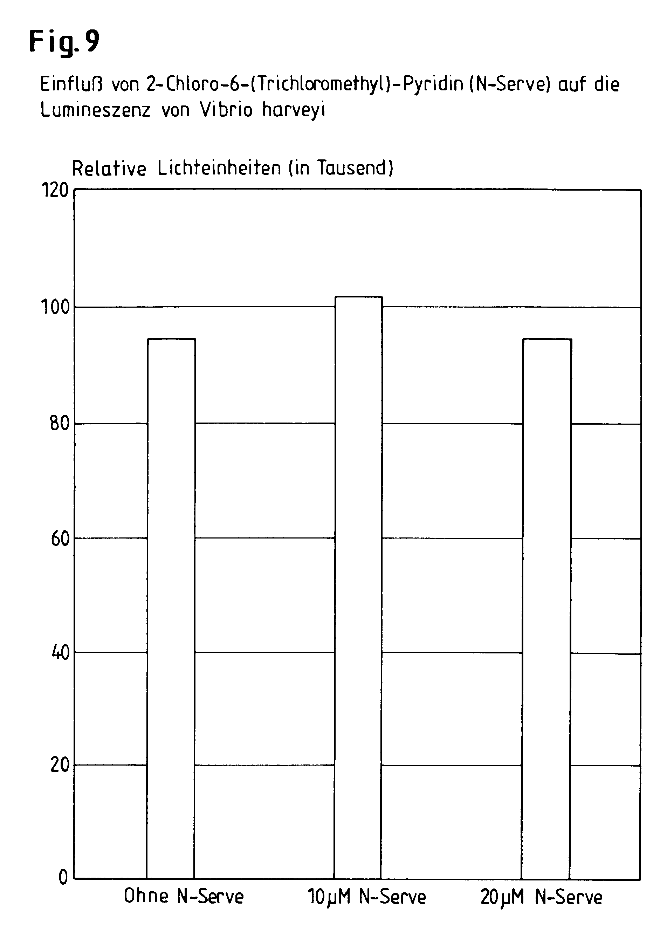

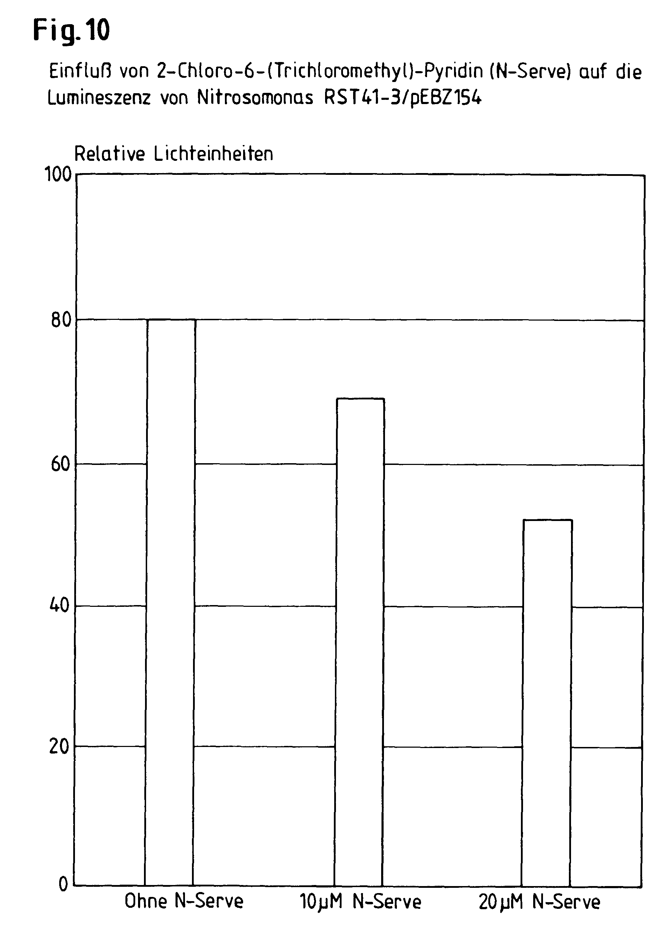

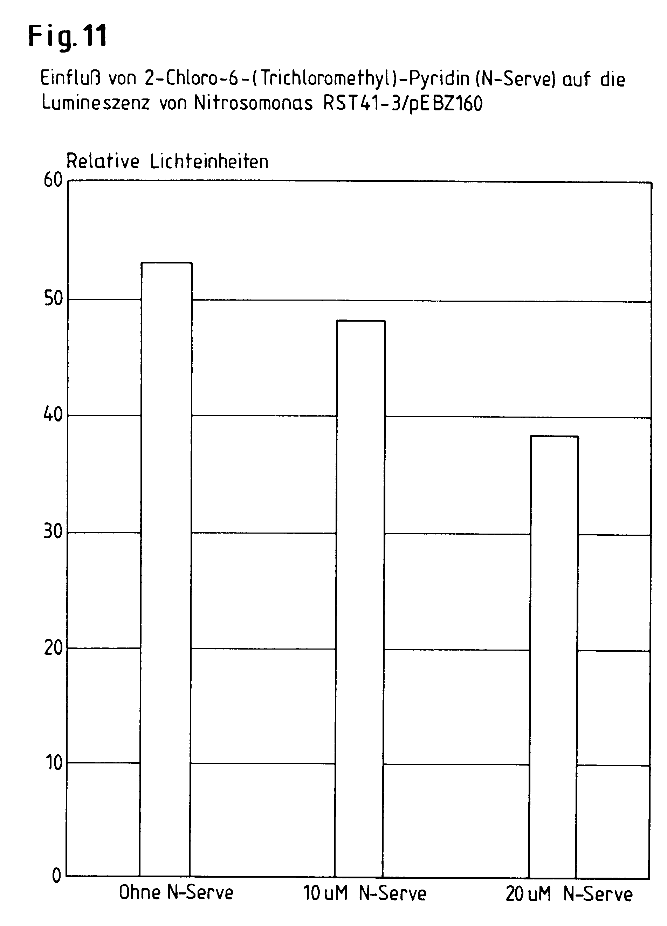

Auch geringe Gaben des Nitrifikationshemmers N-Serve (2-Chloro-6-(Trichloro methyl)-Pyridin) bewirkten lediglich bei den lumineszenten Ammoniakoxidanten *N*. RST41-3/pEBZ154 und *N*. RST41-3/pEBZ160 eine signifikante Verminderung der Leuchtintensität (Abb. 9, 10 und 11).

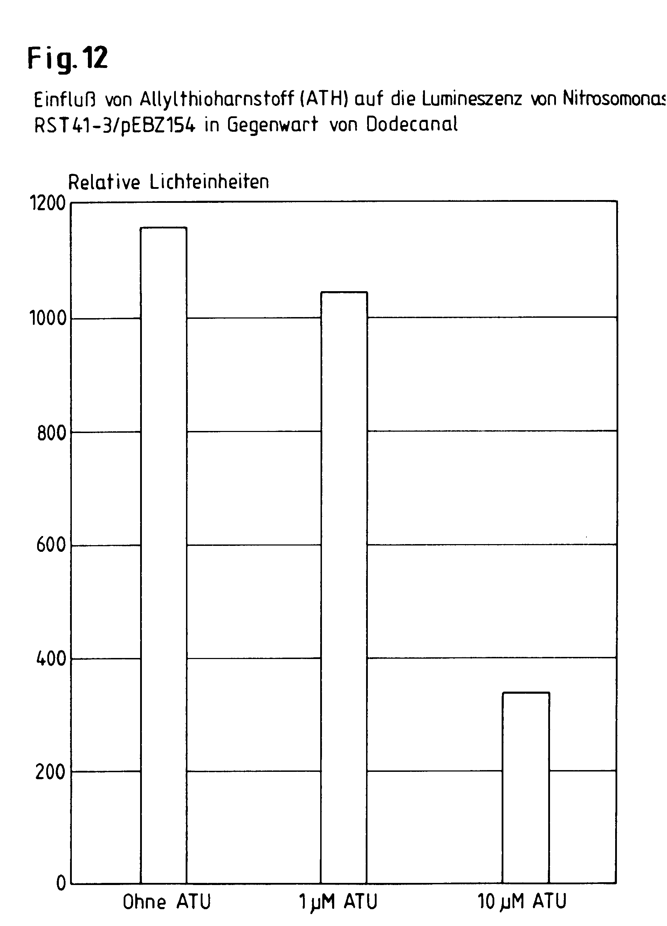

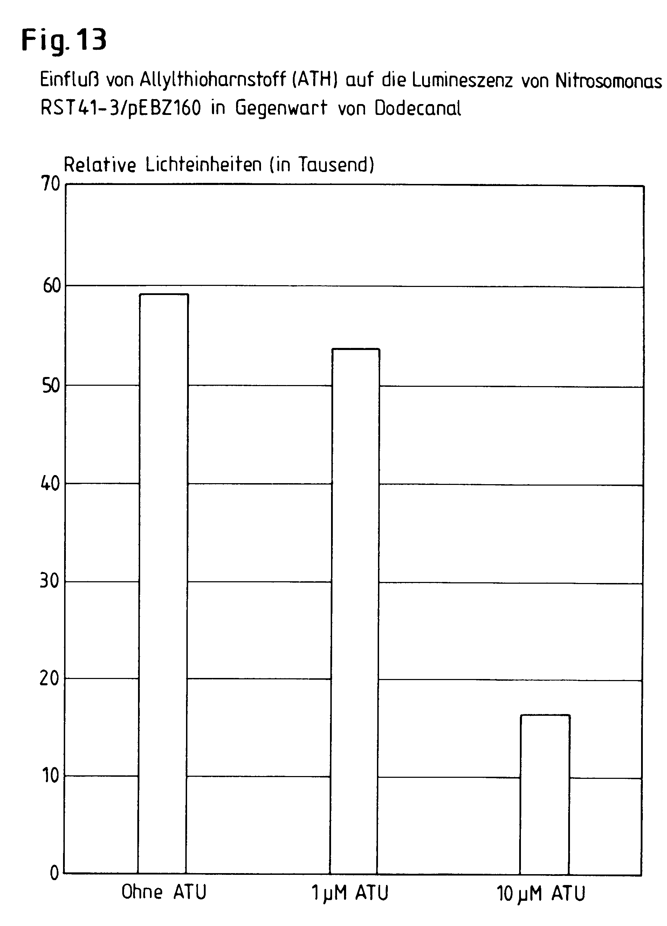

Da bekannt ist, daß sich die Lumineszenz von natürlichen Leuchtbakterien durch Zugabe des Lumineszenz-Substrates, eines langkettigen Aldehyds steigern läßt, wurde Dodecanal ("Bacterial Bioluminescence: Isolation and Expression of the Luciferase Genes from *Vibrio harveyi*", R. Belas, A. Mileham, D. Cohn, M. Hilmen, M. Simon, M. Silverman, Science 218, Seiten 791-793, 1982) dem Testansatz unmittelbar vor den Lumineszenzmessung zugegeben. Auch die gentechnisch veränderten Ammoniakoxidierer reagierten mit einer starken Leuchtzunahme. Diese war jedoch in Gegenwart des Nitrifikationshemmers Allylthioharnstoff drastisch verringert (Abb. 12 und 13).

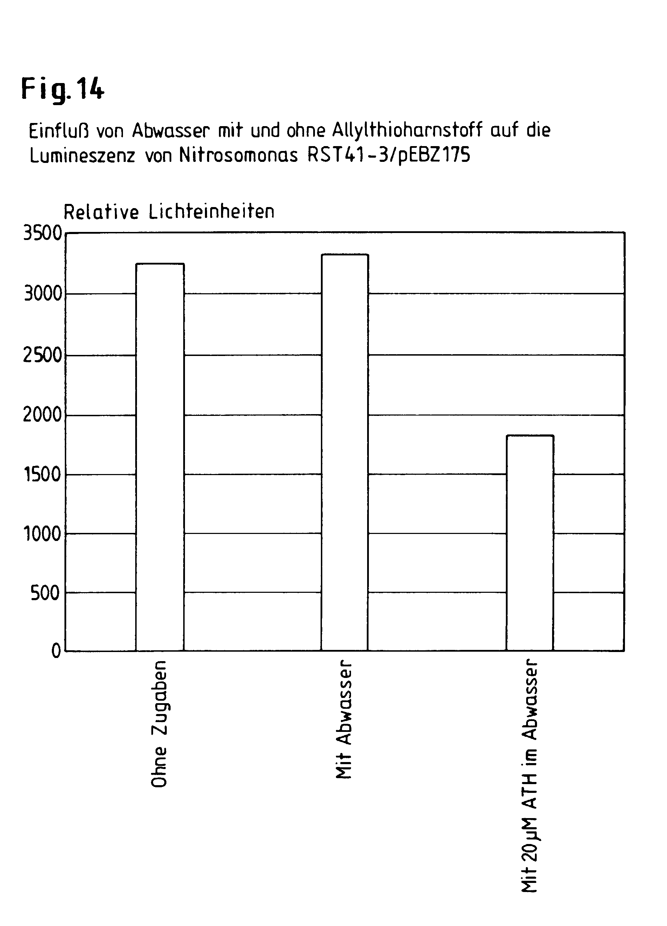

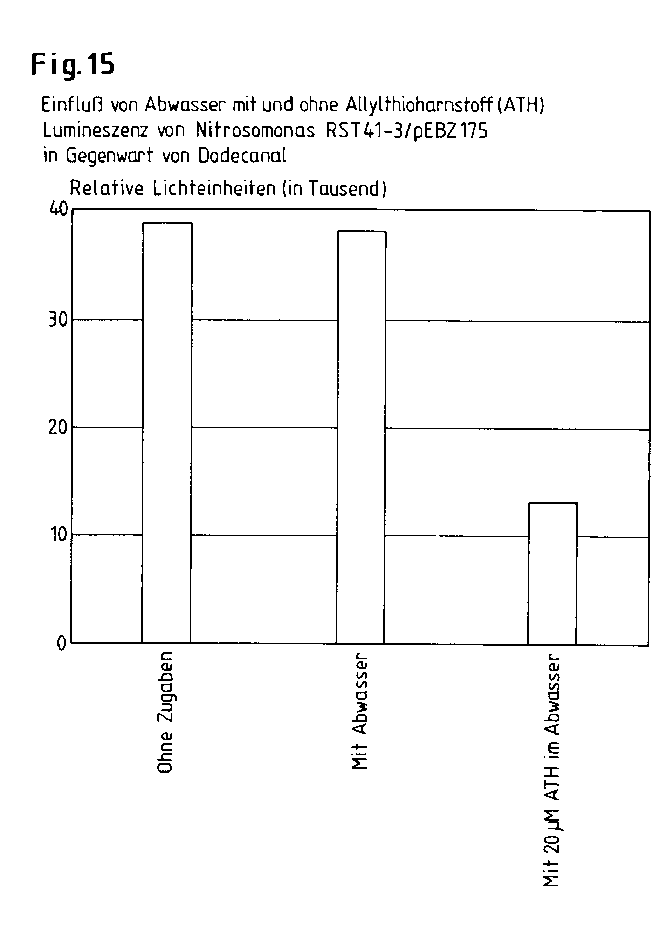

Die Stämme *Nitrosomonas* RST41-3/pEBZ160 und *N*. RST41-3/pEBZ175 wurden daraufhin getestet, die Anwesenheit von Allylthioharnstoff im Ablauf der Kläranlage der Bayer AG in Leverkusen anzuzeigen. Hierbei wurde das Abwasser mit einem Volumen doppelt konzentriertem Medium vermischt. Ein Teil dieser Mischung wurde zu zwei Teilen der Bakteriensuspension zugesetzt. Tatsächlich reagierten beide Stämme nur in Gegenwart von zugesetztem Allylthioharnstoff mit einer drastischen Lumineszenzminderung. Der eingesetzte Klon *Ns*. RST41-3/pEBZ175 wies eine vergleichsweise hohe autonome Lumineszenz auf (Abb. 14). Auch die Lumineszenz dieses Konstruktes ließ sich durch Dodecanal steigern, wobei 20 µM Allylthioharnstoff in der eingesetzten Abwasserprobe ebenfalls zu einer Lumineszenzminderung führten (Abb. 15).

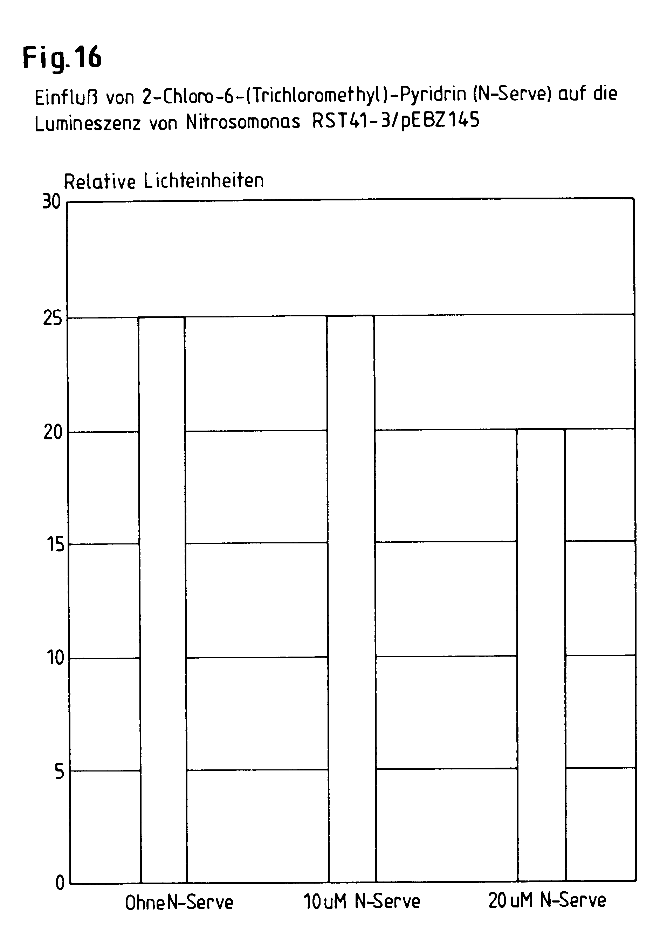

Der Stamm *Ns*. RST41-3/pEBZ145 zeigte nur eine äußerst schwache Lumineszenz, die sich jedoch durch die Gegenwart von 20 µM N-Serve beeinträchtigen ließ (Abb. 16).

### Vorteil des neuentwickelten Testverfahrens zum Nachweis von Hemmstoffen der Nitrifikation

Lumineszente Nitrifizierer eröffnen die Möglichkeit, Hemmstoffe der Nitrifikation selektiv und sensitiv mit einem einfachen automatisierbaren Verfahren zu erkennen. Toxische Stöße eines Nitrifikationshemmstoffes im Abwasser können schnell registriert werden, wodurch sich ein entsprechend großer Zeitraum für Maßnahmen zum Schutz einer Kläranlage ergibt. Darüber hinaus erlauben es die hergestellten Nitrifizierer, den einleitenden Schritt der biologischen Stickstoffeliminierung in Kläranlagen oder anderen komplexen Matrices über eine einfache Messung der Lumineszenz zu untersuchen und zu optimieren. Dies ist insofern möglich, als die Biolumineszenz die Vitalität der Mikroorganismenzelle wiederspiegelt. Mit der gleichen Vorgehensweise sind auch andere biotechnologische Verfahren, an denen chemolithotrophe Bakterien beteiligt sind in analoger Weise zu optimieren. Dabei ist z. B. an ein Leaching-Verfahren zu denken.

## Patentansprüche

1. Verfahren zur Herstellung von biolumineszenten Bakterien, dadurch gekennzeichnet, daß die Erbinformation für das biologische Leuchten auf obligat chemolithoautotrophe nitrifizierende Mikroorganismen übertragen wird.

2. Verfahren nach Anspruch 1, dadurch gekennzeichnet, daß mit Hilfe von Plasmid- oder Transposonvektorsystemen Luciferase-DNA oder eine Luciferase-β-Galactosidase-Transkriptionsfusion in ammoniakoxidierende Bakterien übertragen wird.

3. Verfahren nach Anspruch 2, dadurch gekennzeichnet, daß als Vektorsystem ein in Ammoniakoxidanten replizierbares Plasmid oder ein nicht replizierbares Suizidplasmid oder ein transposables Element verwendet wird.

4. Verfahren nach Anspruch 3, dadurch gekennzeichnet, daß die Herstellung von Nitrosomonas DNA-Fragmenten enthaltenden Vektoren mit den Bezeichnungen pEBZ154 und pEBZ 160 über die nachfolgend definierten Zwischenstufen a) - e) erfolgt:
a) In die *Eco* Rl Restriktionsendonuklease-Schnittstelle des Plasmids pSUP202 wurde die Kanamycinresitenzgen-Kassette aus dem Plasmid pRME1 kloniert. Das resultierende Plasmid erhielt die Bezeichnung pSUP202-1.
b) In das Vektorplasmid pUC18 wurde ein *Bam* Hl-DNA-Fragment aus dem Transposon Tn*4431*, das die Luciferase-(*lux*-) Strukturgene *C, D, A, B, E* und *G* enthält, in die *Bam*HI-Schnittstelle inseriert. Das resultierende Plasmid erhielt die Bezeichnung pEBZ110.
c) In die *Nco* I-Schnittstelle des gemäß Schritt b) hergestellten Plasmids pEBZ110, die im *luxG*-Gen lag, wurde die *lacZ*-Kassette aus dem Plasmid pAB1001 kloniert. Das erzielte Plasmid, das eine *lux-lacZ*-Operonfusion trug, erhielt die Bezeichnung pEBZ119.
d) Die *lux-lacZ*-Fusion wurde aus dem gemäß Schritt c) erhaltenen Plasmid pEBZ119 mit *Bam* Hl herausgeschnitten und in die *Bam* Hl -Schnittstelle des gemäß Schritt a) hergestellten Plasmids pSUP202-1 inseriert. Das so hergestellte Plasmid erhielt die Bezeichnung pEBZ127.
e) In das gemäß Schritt d) hergestellte Plasmid pEBZ127 wurden *Pst* I-Fragmente, isoliert aus der Gesamt-DNA des Bakterienstammes *Nitrosomonas* sp. RST41-3, in die *Pst* I-Schnittstelle kloniert. Die resultierenden, oben angegebenen Vektoren pEBZ154 und pEBZ160 enthalten ein 2.7 kb großes bzw. ein 10 bis 11 kb großes DNA-Fragment.

5. Verfahren nach Anspruch 3 und 4, dadurch gekennzeichnet, daß die Herstellung eines Lumineszenz und β-Galaktosidase Aktivität vermittelnden Vektors mit der Bezeichnung pEBZ175 über die nachfolgend definierten Zwischenstufen a) - e) erfolgt:
a) Der Plasmidvektor pVK102 wurde mit dem Restriktionsenzym *Xho* I-linearisiert und mit dem ebenfalls mit *Xho* I-linearisierten Plasmid pRME1 ligiert. Das resultierende Ligierungsprodukt erhielt die Bezeichnung pVK102-2.
b) Das Ligierungsprodukt pVK102-2 gemäß Schritt a) wurde mit den Enzymen *Bgl* II und *Pst* I geschnitten und das Plasmidreplikon von pVK102 enthaltende DNA-Fragment isoliert und rezyklisiert. Das resultierende Plasmid erhielt die Bezeichnung pEBZ112.
c) In die *Bam* Hl-Schnittstelle des im Schritt b) erhaltenen Plasmid pEBZ112 wurde das *lux-lacZ*-DNA-Fragment aus dem Plasmid pEBZ119 (Anspruch 4, Schritt c)) kloniert. Das resultierende Plasmid erhielt die Bezeichnung pEBZ144.
d) Die im Schritt c) erhaltenen Plasmid pEBZ144 stromabwärts der *lux-lacZ*-Fusion liegende Bam Hl-Schnittstelle wurde aufgefüllt und somit eliminiert. Das so hergestellte Plasmid erhielt die Bezeichnung pEBZ173.
e) In die verbliebene, stromaufwärts der *lux-lacZ*-Fusion liegende *Bam* Hl-Schnittstelle wurde im letzten Schritt zur Herstellung des oben angegebenen Vektors pEBZ175 das ein Neomycinresistenzgen und den *tac*-Promotor tragende *Bam* HI-Fragment aus dem Transposon Tn*5*-B60 so kloniert, daß der tac-Promotor die *lux*- und die *lacZ*-Gene anschaltet.

6. Verfahren nach Anspruch nach Anspruch 3 und 4, dadurch gekennzeichnet, daß die Herstellung eines Transposonvektors mit der Bezeichnung pEBZ177 über die nachfolgend definierten Stufen a) und b) erfolgt:
a) Das Neomycinresistenzgen aus Tn*5*-B60 wurden mit dem Restriktionsenzym *Bam* HI deletiert. In die *Bgl* II-Schnittstelle dieses Deletionsproduktes wurde die Kanamycinresistenzgen-Kassette aus dem Plasmid pRME1 inseriert. Das resultierende Plasmid erhielt die Bezeichnung pEBZ140.
b) In die *Bam* Hl-Schnittstelle des in Schritt a) erhaltenen Plasmids pEBZ140 wurde zur Herstellung des oben angegebenen Transposon-tragenden Vektors pEBZ177 das eine *lux-lacZ*-Fusion tragende *Bam* Hl-DNA-Fragment des Plasmides pEBZ119 (Anspruch 4, Schritt c)) kloniert.

7. Verfahren nach Anspruch 1 - 6, dadurch gekennzeichnet, daß ein Vektor verwendet wird, der als Reportergen aus eine lux-DNA oder eine lux-lacZ Operonfusion enthält.

8. Verfahren nach Anspruch 1 - 7, dadurch gekennzeichnet, daß als ammoniakoxidierende Bakterien Nitrosomonasstämme, insbesondere RST41-3 eingesetzt werden.

9. Biolumineszente nitrifizierende Mikroorganismen erhältlich nach den Verfahren gemäß Anspruch 1 - 8.

10. Biolumineszente nitrifizierende Mikroorganismen nach Anspruch 9, gekennzeichnet durch gentechnisch modifizierte obligat chemolithoautotrophe Ammoniakoxidanten.

11. Biolumniszente nitrifizierende Mikroorganismen nach Anspruch 10, gekennzeichnet durch gentechnisch modifizierte Nitrosomonasstämme.

12. Biolumineszente nitrifizierende Mikroorganismen nach Anspruch 11 und nach Anspruch 5 oder 6, gekennzeichnet durch Abkömmlinge von Nitrosomonas RST41-3, die durch Transfer des Plasmids pEBZ175 oder des Transposon-tragenden Plasmids pEBZ177 gebildet wurden.

13. Biolumnineszente nitrifizierende Mikroorganismen nach Anspruch 8 - 12, gekennzeichnet durch den Stamm RST41-3/pEBZ154 gemäß Hinterlegungsnummer DSM 9146.

14. Verfahren zur Messung der Vitalität von Mikroorganismen durch Messung der Abnahme der Lumineszenz, dadurch gekennzeichnet, daß Mikroorganismen gemäß Anspruch 9 - 13, verwendet werden.

15. Verfahren nach Anspruch 14, dadurch gekennzeichnet, daß die biolumineszenten Mikroorganismen bei der Nitrifikation als Indikator für evt. vorhandene Hemmstoffe eingesetzt werden.

16. Verfahren nach Anspruch 15, dadurch gekennzeichnet, daß Hemmstoffe in einer nitrifizierenden biologischen Kläranlage detektiert werden.

## Claims

1. A method of producing bioluminescent bacteria, characterised in that the genetic information for biological luminescence is transferred to obligatorily chemolithoautotrophic nitrifying microorganisms.

2. A method according to claim 1, characterised in that luciferase DNA or a luciferase-β-galactosidase transcription fusion is transferred into ammonia-oxidising bacteria with the aid of plasmid- or transposon vector systems.

3. A method according to claim 2, characterised in that a plasmid which is replicable in ammonia oxidants or a non-replicable suicide plasmid or a transposable element is used as the vector system.

4. A method according to claim 3, characterised in that production of the vectors with the designations pEBZ154 and pEBZ160, which contain *Nitrosomonas* DNA fragments, is effected via intermediate steps a) - e) as defined below:
a) the kanamycin resistance gene cassette from the plasmid pRME1 was cloned into the *Eco* RI restriction endonuclease cleavage site of the plasmid pSUP202. The resulting plasmid was given the designation pSUP202-1;
b) α *Bam* HI-DNA fragment from the transposon Tn*4431*, which contains the luciferase-(lux) structural genes *C*, *D, A, B, E* and *G*, was inserted in the *Bam* HI cleavage site in the vector plasmid pUC18. The resulting plasmid was given the designation pEBZ110;
c) the *lacZ* cassette from the plasmid pAB1001 was cloned into the *Nco* I cleavage site of plasmid pEBZ110 produced according to step b), which was situated in the *lux*G gene. The plasmid obtained, which comprised a *lux-lac*Z operon fusion, was given the designation pEBZ119;
d) the *lux-lac*Z fusion was cut with *Bam* HI from plasmid pEBZ119 obtained according to step c) and was inserted in the *Bam* HI cleavage site of plasmid pSUP202-1 produced according to step a). The plasmid produced in this manner was given the designation pEBZ127;
e) *Pst* I fragments, isolated from the total DNA of the bacterial strain *Nitrosomonas* sp. RST41-3, were cloned into the *Pst* I cleavage site in plasmid pEBZ127 produced according to step d). The resulting vectors pEBZ154 and pEBZ160 given above contain a DNA fragment of size 2.7 kb and a DNA fragment of size 10 to 11 kb, respectively.

5. A method according to claims 3 or 4, characterised in that the production of a vector with the designation pEBZ175, which relays luminescence and β-glactosidase activity, is effected via intermediate steps a) - e) as defined below:
a) the plasmid vector pVK102 was linearised with the restriction enzyme *Xho* I and was ligated with the plasmid pRME1, which was likewise linearised with *Xho* I. The resulting ligation product was given the designation pVK102-2;
b) the ligation product pVK102-2 according to step a) was cut with the enzymes *Bgl* II and *Pst* I and the DNA fragment containing the plasmid replicon of pVK102 was isolated and recycled. The resulting plasmid was given the designation pEBZ112;
c) the *lux-lac*Z DNA fragment from the plasmid pEBZ119 (claim 4, step c)) was cloned into the *Bam* HI cleavage site of plasmid pEBZ112 obtained in step b). The resulting plasmid was given the designation pEBZ144;
d) the *Bam* HI cleavage site situated downstream of the *lux-lac*Z fusion in plasmid pEBZ144 obtained in step c) was filled and was thus eliminated. The plasmid produced in this manner was given the designation pEBZ173;
e) in the final step, for the production of the aforementioned vector pEBZ175, the *Bam* HI fragment from the transposon Tn*5*-B60, which fragment carried a neomycin resistance gene and the *tac* promoter, was cloned into the remaining *Bam* HI cleavage site situated upstream of the *lux-lac*Z fusion so that the *tac* promoter may switch on the *lux* and *lac*Z genes.

6. A method according to claims 3 or 4, characterised in that the production of a transposon vector with the designation pEBZ177 is effected by way of steps a) and b) as defined below:
a) the neomycin resistance gene from Tn5-B60 was deleted with the restriction enzyme *Bam* HI. The kanamycin resistance gene from the plasmid pRME1 was inserted in the *Bgl* II cleavage site of this deletion product. The resulting plasmid was given the designation pEBZ140;
b) for the production of the aforementioned transposon-carrying vector pEBZ177, the *Bam* HI DNA fragment of plasmid pEBZ119 (claim 4, step c)), which fragment carried a *lux-lac*Z fusion, was cloned into the *Bam* HI cleavage site of plasmid pEBZ140 obtained in step a).

7. A method according to claims 1 - 6, characterised in that a vector is used which contains a *lux*-DNA or a *lux-lac*Z operon fusion as a reporter gene.

8. A method according to claims 1 - 7, characterised in that *Nitrosomonas* strains, particularly RST41-3, are used as the ammonia-oxidising bacteria.

9. Bioluminescent nitrifying microorganisms obtainable by the method according to claims 1 - 8.

10. Bioluminescent nitrifying microorganisms according to claim 9, characterised by obligatorily chemolithoautotrophic ammonia oxidants modified by genetic engineering.

11. Bioluminescent nitrifying microorganisms according to claim 10, characterised by *Nitrosomonas* strains modified by genetic engineering.

12. Bioluminescent nitrifying microorganisms according to claim 11 and according to claims 5 or 6, characterised by derivatives of *Nitrosomonas* RST41-3 which are formed by transfer of the plasmid pEBZ175 or of the transposon-carrying plasmid pEBZ177.

13. Bioluminescent nitrifying microorganisms according to claims 8 - 12, characterised by the strain RST41-3/pEBZ154 according to deposition number DSM 9146.

14. A method of measuring the vitality of microorganisms by measuring the decrease in luminescence, characterised in that microorganisms according to claims 9 - 13 are used.

15. A method according to claim 14, characterised in that the bioluminescent microorganisms are used during nitrification as an indicator for inhibitors which are possibly present.

16. A method according to claim 15, characterised in that inhibitors are detected in a nitrifying biological sewage treatment plant.

## Revendications

1. Procédé de préparation de bactéries bioluminescentes, caractérisé en ce que l'information héréditaire pour la luminescence biologique est transférée à des micro-organismes nitrifiants, obligatoirement chimiolithoautotrophes.

2. Procédé selon la revendication 1, caractérisé en ce qu'un ADN de luciférase ou une fusion de transcription luciférase-β-galactosidase est transféré dans des bactéries oxydant l'ammoniaque, au moyen de systèmes vecteurs plasmidiques ou par transposon.

3. Procédé selon la revendication 2, caractérisé en ce que l'on utilise comme système vecteur, un plasmide réplicable dans les oxydants de l'ammoniaque ou un plasmide suicide non réplicable ou un élément transposable.

4. Procédé selon la revendication 3, caractérisé en ce que la préparation de vecteurs contenant des fragments d'ADN de Nitrosomonas, nommés pEBZ154 et pEBZ160, se fait par les étapes intermédiaires a) - e), définies dans ce qui suit :
a) On clone la cassette du gène de résistance à la kanamycine provenant du plasmide pRME1 au site de restriction de l'endonucléase de restriction Eco RI du plasmide pSUP202. Le plasmide résultant est nommé pSUB202-1.
b) Dans le plasmide vecteur pUC18, on insère au site de restriction Bam HI, un fragment d'ADN Bam HI provenant du transposon Tn4431, qui contient les gènes de structure C, D, A, B, E et G de la luciférase (lux) Le plasmide résultant est nommé pEBZ110.
c) Au site de restriction Nco I du plasmide pEBZ110 préparé suivant l'étape b), qui se trouve dans le gène luxG, on clone la cassette lacZ du plasmide pAB1001. Le plasmide obtenu, qui porte une fusion lux-opéron lacZ, est nommé pEBZ119.
d) La fusion lux-lacZ est prélevée au moyen de Bam HI du plasmide pEBZ119 obtenu suivant l'étape c) et insérée au site de restriction Bam HI du plasmide pSUP202-1 préparé suivant l'étape a). Le plasmide ainsi préparé est nommé pEBZ127.
e) Dans le plasmide pEBZ127 préparé suivant l'étape d), on clone le fragment Pst I, isolé de l'ADN complet de la souche bactérienne Nitrosomonas sp. RST41-3, au site de restriction Pst I. Les vecteurs pEBZ154 et pEBZ160 résultants, indiqués ci-dessus, contiennent un fragment d'ADN de 2,7 kb et respectivement, de 10 à 11 kb.

5. Procédé selon les revendications 3 et 4, caractérisé en ce que la préparation d'un vecteur fournissant la luminescence et l'activité β-galactosidase, nommé pEBZ175, se fait par les étapes intermédiaires a)-e) définies dans ce qui suit :
a) Le vecteur plasmide pVK102 est linéarisé avec l'enzyme de restriction Xho I et ligaturé au plasmide pRME1 également linéarisé par Xho I. Le produit de ligature résultant est nommé pVK102-2.
b) Le produit de ligature pVK102-2 suivant l'étape a) est coupé par les enzymes Bgl II et Pst I et le fragment d'ADN contenant le réplicon plasmidique de pVK102 est isolé et recyclisé. Le plasmide résultant est nommé pEBZ112.
c) Au site de restriction Bam HI du plasmide pEBZ112 obtenu à l'étape b), on clone le fragment d'ADN lux-lacZ provenant du plasmide pEBZ119 (revendication 4, étape c). Le plasmide résultant est nommé pEBZ144.
d) Le site de restriction Bam HI se trouvant en aval de la fusion lux-lacZ dans le plasmide pEBZ144 obtenu à l'étape c) est complété et ainsi éliminé. Le plasmide ainsi préparé est nommé pEBZ173.
e) Au site de restriction Bam HI maintenu, se trouvant en amont de la fusion lux-lacZ, on clone, dans la dernière étape de préparation du vecteur pEBZ175 indiqué ci-dessus, le fragment Bam HI portant un gène de résistance à la néomycine et le promoteur tac, provenant du transposon Tn5-B60, de sorte que le promoteur tac soit au voisinage proche des gènes lux et lacZ.

6. Procédé suivant les revendications 3 et 4, caractérisé en ce que la préparation d'un vecteur transposon nommé pEBZ177 se fait par les étapes a) et b) définies dans ce qui suit :
a) Le gène de résistance à la néomycine provenant de Tn5-B60 est délété par l'enzyme de restriction Bam HI. Au site de restriction Bgl II de ce produit de délétion, on insère la cassette du gène de résistance à la kanamycine provenant du plasmide pRME1. Le plasmide résultant est nommé pEBZ140.
b) Au site de restriction Bam HI du plasmide pEBZ140 obtenu à l'étape a), on clone, pour la préparation du vecteur pEBZ177 portant le transposon, indiqué ci-dessus, le fragment d'ADN Bam HI portant une fusion lux-lacZ du plasmide pEBZ119 (revendication 4, étape c).

7. Procédé selon les revendications 1 à 6, caractérisé en ce que l'on utilise un vecteur qui contient comme gène reporter, un ADN lux ou une fusion lux-opéron lacZ.

8. Procédé selon les revendications 1 à 7, caractérisé en ce que l'on introduit comme bactéries oxydant l'ammoniaque, des souches de Nitrosomonas, en particulier RST41-3.

9. Micro-organismes nitrifiants et bioluminescents, pouvant être obtenus par le procédé selon les revendications 1 à 8.

10. Micro-organismes nitrifiants et bioluminescents selon la revendication 9, caractérisés en ce qu'ils sont oxydants de l'ammoniaque, obligatoirement chimiolithoautotrophes et modifiés par génie génétique.

11. Micro-organismes nitrifiants et bioluminescents selon la revendication 10, caractérisés en ce qu'ils sont des souches de Nitrosomonas modifiées par génie génétique.

12. Micro-organismes nitrifiants et bioluminescents selon la revendication 11 et selon la revendication 5 ou 6, caractérisés en ce qu'ils sont des dérivés de Nitrosomonas RST41-3, qui sont formés par transfert du plasmide pEBZ175 ou du plasmide portant un transposon pEBZ177.

13. Micro-organismes nitrifiants et bioluminescents selon les revendications 8 à 12, caractérisés par la souche RST41-3/pEBZ154 suivant le numéro de dépôt DSM 9146.

14. Procédé pour mesurer la vitalité de micro-organismes par la mesure de la diminution de la luminescence, caractérisé en ce que l'on utilise des micro-organismes selon les revendications 9 à 13.

15. Procédé selon la revendication 14, caractérisé en ce que l'on introduit les micro-organismes bioluminescents lors de la nitrification, comme indicateur d'inhibiteurs éventuellement présents.

16. Procédé selon la revendication 15, caractérisé en ce que les inhibiteurs sont détectés dans un dispositif de clarification biologique nitrifiante.
